# EUROPEAN PATENT APPLICATION

(11) **EP 2 764 847 A1**
(43) Date of publication of application: **13.08.2014**
(21) Application number: 13154604.6
(22) Date of filing: 08.02.2013
(51) Int. Cl.: A61F 2/26

(54) **Penile augmentation**

(71) Applicant: Kirch Urologie B.V., 2313 EW Leiden (NL)
(72) Inventor: Kirch, Joannes Antonius Jules Marie, 2313 EW Leiden (NL)
(74) Representative: Algemeen Octrooi- en Merkenbureau B.V.

(57) **Abstract**

The present invention relates to a penile implant. The present invention further relates to a method for manufacturing a penile implant. The invention further relates to a kit of a number of penile implants. The invention further relates to a penile implant or kit for use in cosmetic treatments.. The invention further relates to a penile implant or kit for use in the prevention of the retraction of the corpora cavernosa during implant of inflatable penile prostheses for erectile dysfunction and expected shortening of penile length during surgical correction of penile curvature like in Nesbitt procedures.

## Description

The present invention relates to a penile implant. The present invention further relates to a method for manufacturing a penile implant. The invention further relates to a kit of a number of penile implants. The invention further relates to a penile implant or kit for use in cosmetic treatments. The invention further relates to a penile implant or kit for use in the prevention of the retraction of the corpora cavernosa.

Penile implants are also known in the art. For example US5,445,594 discloses an implant device for expanding the girth and length of a penis. A soft, flexible body is implanted between the shaft and the skin of the penis. The body takes the shape of a partial cylindrical sleeve that has an outer, relatively elastic sheet member and an inner, relatively inelastic sheet member. When implanted, the body covers the corpus cavernosum of the penis and does not or only partially covers the urethra, and extends in length between the glans penis and the base of the penis. A principally closed sack is formed between the inner and outer sheet members for receiving a fluid under pressure from a fluid source. Spring-like ribs are embedded within the inner sheet member for preventing collapse of the inner sheet member when the body is deflated. Implanting this implant involves a relative invasive procedure and operating the implant is also bothersome and involves injecting liquids into a receiver of the implant resulting in possible dangerous leakage of fluid outside the implant into the body via lymph- and/or bloodvessels.

A further implant is disclosed in WO03/103537 which discloses a surgically implanted penile prosthesis, including an apical prosthetic element to be implanted as a pad in a sub-glans position, between the distal surface of the corpora cavernosa and the glans , such apical prosthetic element being of suitable size and shape to result, upon implant, in an overall increase in glans volume with no substantial alterations to the typical anatomical shape of the glans itself.

Penile implants are also used to solve erectile problems. For example, US2012/053400 discloses a body implantable penile prosthetic assembly which includes a pump and a release mechanism. The pump is connectable to a pressure reservoir and a penile implant, where the pressure reservoir contains a pressurized NaCl 0,9% liquid at a first pressure when implanted. The release mechanism is connectable between the pressure reservoir and the penile implant and is configured to release the pressurized liquid from the pressure reservoir to inflate the penile implant to a second pressure that is less than the first pressure. The pump is operable to transfer the pressurized liquid from the penile implant to the pressure reservoir to deflate the penile implant, and pressurize the liquid in the pressure reservoir from the second pressure to at least the first pressure. After implant of implants to solve erectile problems shortening of the penis can occur.

At present penile lengthening procedures can be performed without implanting an implant. In such a procedure the three suspensory ligaments are cut. The suspensory ligaments connect the dorsal corpora cavernosa with the pubic symphysis. By cutting the ligaments an increase in length can be obtained since the parts of the dorsal corpora cavernosa disconnected from the pubic symphysis will contribute to the length of the penis. In this prior art method infrapubic naturally present fat can be used to be sutured in the created cavity to prevent the direct retraction of the freed proximal corpora cavernosa . The disadvantage of this procedure is that approximately 25% of the patients which have undergone this procedure postoperative shortening occurs partly or completely undoing the gained increase in penile length. This shortening is caused by fibrosis and necrosis of the fat deposited in the created cavity.

It is an object of the present invention to overcome the problem of loss of penile length after a penile lengthening or correction procedure.

It is an object of the present invention to provide for an implant not having the disadvantage of penile shortening after implant.

It is an object of the present invention to provide for a penile implant that overcomes the problem of postoperative shortening of the penis after penile operation such as penile lengthening procedures, implantation of an implant overcoming erectile dysfunction and correction of the absolutely to be expected penile shortening in penile curvature surgery like the Nesbitt procedure.

One or more of the objects is achieved by an implant according to the preamble having at least a first and second side wherein when implanted the first side face the pubic symphisis and the second side faces the dorsal corpora cavernosa. The implant according to the present invention is placed in the cavity obtained after cutting the three suspensory ligaments disconnecting the dorsal corpora cavernosa from the pubic symphisis. The implant is orientated in the body in such a way that a first side of the implant faces the pubic symphisis and a second side of the implant faces the dorsal corpora cavernosa. This prevents fibrosis at the site and hence prevents retraction of dorsal corpora cavernosa and thus prevents postoperative shortening.

Said second side is preferably provided with 2 cavities for receiving the corpora cavernosa when implanted in the body. These cavities preferably extend along the complete length of the implant according to the present invention.

In an embodiment of the present invention the penile implant is substantially triangular in shape. The triangular shape allows for the base of the penis to be shaped in a natural way. Further this shape allows for the base of the penis to be broadened. The size (its girth) of the base of the penis is known to stimulate the clitoris during sexual intercourse. Advantageously broadening of the base will result in an increased stimulation of the clitoris during sexual intercourse. Hence the invention further provides for a method of increasing the girth of the penis by implanting an implant according to the present invention, preferably the method increases the girth by 2-3cm.

Preferably, the first and second side define an angle, said angle preferably is from 45 to 75 degrees. Preferably, the implant according to the present invention has comprises an edge which spans between the first and second side. The edge and the first and second side define two planes. Said planes are enclosed by the edge, the first and second side.

In an embodiment of the present invention the penile implant comprises means to connecting the implant to at least the pubic symphisis and/or the corpora cavernosa. These means allow for the implant to be connected to the pubic symphisis and/or the corpora cavernosa in order for the implant to be kept in place until fibrous capsular formation around the implant is completed. Preferably said means for connecting is a mesh-work of fibers. Preferably said mesh-work is comprised of polypropylene. The mesh work allows for the implant to be fixed to the pubic symphisis and/or the corpora cavernosa by stiches. Preferably said means are at least present on the first and second side of the implant. Both lateral sides of the implant will contain a band of mesh fibers to connect those sides to the lateral remnants of the cut suspensory ligaments for extra position stability in the time needed for definitive fibrous capsular formation around the implant. In a further embodiment of the present invention the mesh work envelopes said solid body of soft medical silicon.

In an embodiment of the present invention said mesh work is embedded superficially in the implant or superficially connected strong enough to the surface of the implant to secure the fixation sutures long enough to be sure the implant will stay in position until fibrous capsular surrounding of the implant has been completed and definitive desired position is to be secured.

In an embodiment of the present invention the penile implant is comprised of soft silicon, and preferably medical grade soft silicon. The soft silicon is preferably of the long-term implantable soft silicon type. Said material is known in the art of medicine and is known as a material suited for implants. It also allows for easy manufacturing of solid body implants. Preferably said implant comprises a solid body of soft silicon.

In an embodiment of the present invention the penile implant consists of a solid body of soft silicon and a mesh work. Such an implant can be manufactured cost efficient and on an industrial scale and via automated methods.

In an embodiment of the present invention the penile implant has a first side ranging from 3 to 5 cm. The first side faces the pubic symphisis when implanted in the body. In order for the implant to cover a maximum of the pubic symphisis sufficient for it to be placed in a body of a person having an average sized pubic symphisis.

In an embodiment of the present invention the penile implant has a second side ranging from 4 to 11 cm. The second side faces the dorsal corpora cavernosa when implanted in the body. In cases of smaller or larger pubic symphisis an implant can be manufactured with customized dimensions.

The present invention further provides for a method of increasing penis length by providing an implant as described above to a body. Said implant is provided to the cavity between the dorsal corpora cavernosa and the pubic symphisis after cutting of the three suspensory ligaments. Said method provides for the same advantages as described above for the implant according to the present invention.

The present invention further relates to a kit comprising a number of penile implants, said penile implants are preferably according to the invention. Preferably said number of penile implants are of different size. Such a kit allows for a surgeon performing an implant procedure to select an implant from the kit with the most suitable dimensions for the patient. This selection can take during the implant procedure at a point in the procedure when it is possible for the surgeon to assess which implant from the kit has the dimensions best suited for the patient. Preferably, the kit according to the present invention comprises at least 3 penile implants, and more preferred at least 6 penile implants.

Said implants are preferably packed in transparent plastic and paper on the backside to easily take out the implant of the surgeons choice during surgery.

Method for manufacturing a penile implant comprising the steps of:
i) Providing a mold;
ii) Providing to the mold soft silicon, medical grade soft solid silicon;
iii) Separating said mold from the silicon after a certain amount of time to obtain a penile implant.

The certain amount of time will depend on the time it takes for the soft silicon to solidify. The method according to the present invention allows for the manufacturing of a solid body silicon penile implant having the advantages as described above.

In an embodiment of the present invention, the method further comprises the step of providing to the implant obtained in step iii) a mesh-work of fibers, preferably said mesh work is in the form of a sheet or tape. This allows for the mesh-work to be positioned on the outside of the implant.

In an embodiment according to the present invention the mesh-work is provided to the mold prior to step ii). This allows for the mesh-work to end up being embedded superficially in the implant.

The present invention relates to a penile implant or a kit comprising a certain number of penile implants for use in a cosmetic treatment. Said penile implant and kit are preferably as described above. Said implant and kit can advantageously be used in a cosmetic treatment and preferably in a treatment for penile lengthening or penile correction. With penile correction straightening of the penis is included. Straightening of a curved penis can for example be achieved via a Nesbitt procedure. The Nesbitt procedure can result I a loss of penile length.

The present invention relates to a penile implant or a kit comprising a certain number of penile implants for use in the prevention of the retraction of the corpora cavernosa. Preferably said implant or kit are according to the present invention and are used in penile lengthening procedures. Said penile implant and kit are preferably as described above.

The present invention relates to a penile implant or a kit for use in combination with a prosthesis for penile erection. With a prosthesis for penile erection is meant a device to be implanted in a person having erectile dysfunction in order to mechanically provide for an erection. Preferably, said implant or kit is used in procedures for implanting devices to overcome the problem of erectile dysfunction. These kinds of procedures are known to also cause shorter penile length in full erection after the procedure. Hence in order to prevent the disappointing maximal penile length in erectile state after insufflation of the prosthesis in the corpora cavernosa, a penile implant or kit according to the present invention can be used.

It is to be noted that depending on the ethnicity or wishes (such as a broader base of the penis) of the recipient kits comprising different sets of implant sizes or shapes can be manufactured.

The figures depict an embodiment according to the present invention and should not be construed as limiting the present invention.
Figure 1 shows a side view of a penis prior to penile augmentation.
Figure 2 shows a side view of a penis after penile augmentation.
Figure 3 shows an intersection of a penis after penile augmentation.
Figure 4 schematically depicts the suspensory ligaments, dorsal corpora cavernosa, the pubic symphysis and the implant.
Figure 5 shows an implant.

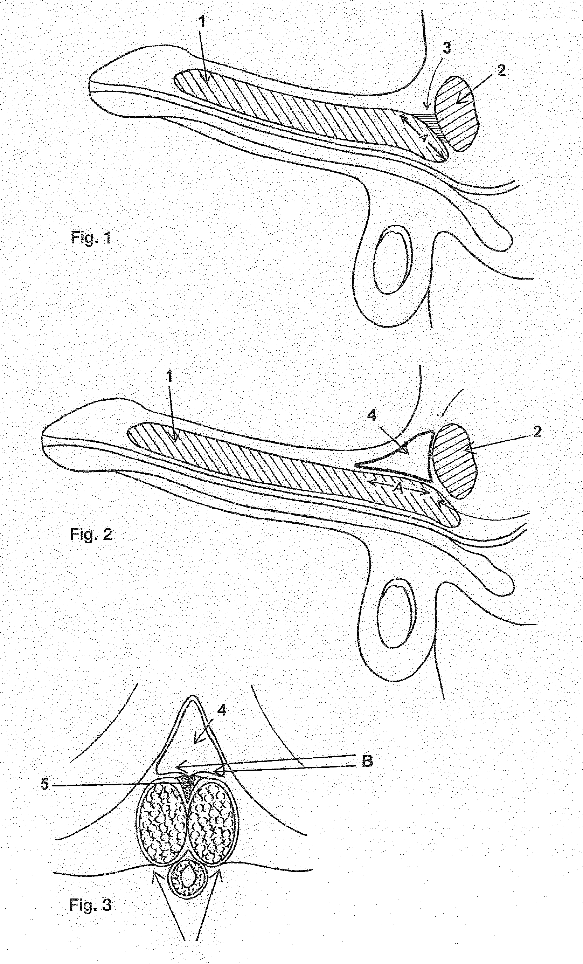

Figure 1 shows a side view of a penis with the suspensory ligaments (3) connecting the dorsal corpora cavernosa (1) with the pubic symphysis (2).
Figure 2 shows a side view of a penis after penile augmentation. In figure 2 an implant (4) according to the present invention has been implanted at the base of the penis. As shown the suspensory ligaments (3) have been cut. The implant (4) is positioned such that one side of the implant (4) faces the dorsal corpora cavernosa (1) and another side the pubic symphysis (2). The length increase is indicated by A.
Figure 3 shows an intersection of a penis after penile augmentation wherein the side of the implant (4) facing the pubic symphysis is facing the viewer. Figure 3 shows how the implant is positioned to cover the neurovascular bundle (5). Further the side of the implant (4) facing the corpora cavernosa (1) is shaped such that it follows the contours of the corpora cavernosa (1). In the implant according to figure 3 this is achieved by providing to cavities (B) to the side facing the corpora cavernosa (1).

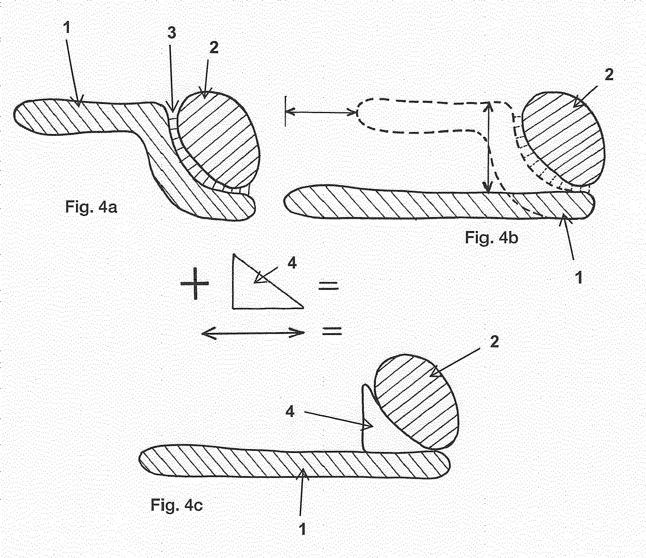

Figure 4a schematically shows the suspensory ligaments (3), connecting the dorsal corpora cavernosa (1) with the pubic symphysis (2). Figure 4b schematically shows the effect of cutting the suspensory ligaments (3). The length over which the corpora cavernosa (1) is separated from the pubic symphysis (2) allows for the penis to be extended This extension is possible since the corpora cavernosa (1) can be moved outward (compared to the body) . The separation also provides for a cavity between (at least) part of the corpora cavernosa (1) and the pubic symphysis (2). In said cavity the implant (4) is placed and connected to the pubic symphysis (2). This prevents the corpora cavernsosa (1) to move back towards its original position (of figure 4a). A more detailed description of a method for implanting an implant according to the present invention is provided further below.

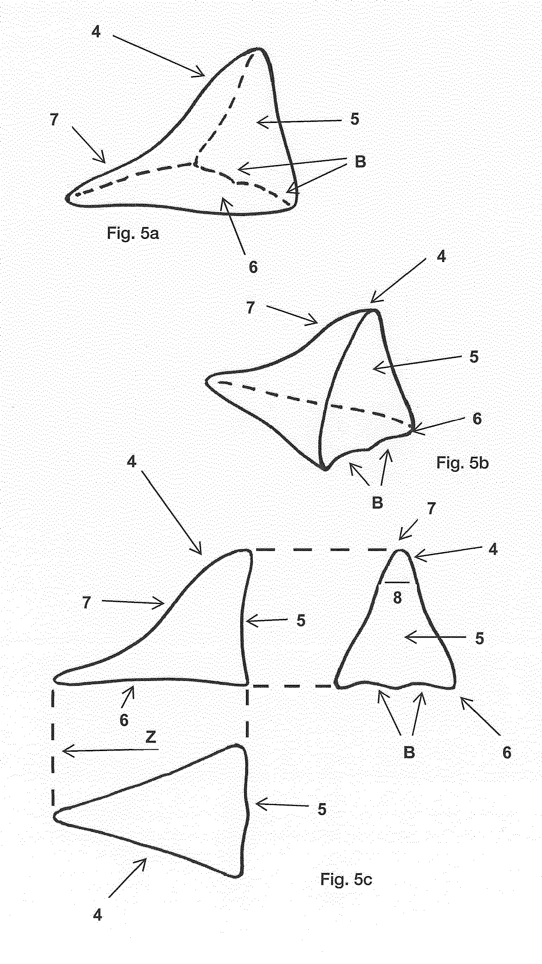

Figures 5a, 5b and 5c show an implant (4) according to an embodiment of the present invention. The implant (4) comprises a first (6) and second side (5) wherein when implanted the first side (5) faces the pubic symphisis and the second side (6) faces the dorsal corpora cavernosa. In figure 5 the second side (6) is provided with two cavities (B) which can receive the corpora cavernosa when implanted. The first side (5) can be curved to follow the contours of the pubic symphisis when implanted.
Figure 5c depicts three views of an implant (4) according to the present invention. The right drawing shows the implant from the second side (5) view. This clearly shows the substantially triangular shape of the implant (4) and further shows the cavities (B) at the first side of the implant. The left top drawing of figure 5c shows a side view of the implant (4). This drawing shows a possible shape of the implant in the direction (indicated by arrow Z) extending from the pubic symphisis when implanted. The bottom drawing of figure 5c depicts the implant (4) from the top side wherein the first side (6) faces away from the viewer.
Figures 5a, 5b and 5c further show edge 7 having an angle 8. Angle 8 can vary along direction Z and can vary from a rounded edge to a sharp V shaped edge. Edge 7 together with the first (6) and second (5) side define two planes making up the side walls of the implant (4). These side walls make up the sides of the implant (4) which do not face the corpora cavernosa or the pubic symphisis when implanted.

Further the decline of edge 7 along direction Z and/or its curvature will depend largely on the shape desired by the recipient. In order to provide for a broader base of the penis edge 7 can be shaped such that it provides for said broadening. Hence the shape of edge 7 (when viewed from outside of the implant) can vary from convex to concave along direction Z , or is completely concave or convex. Edge 7 can also be straight.

### A method for implanting

A patient is treated after approval of the anesthesist with spinal, epidural or general anesthesia on a certified Class 1 OR under antibiotic prophylactic regimen. V-incision of 5cm on the lower side of the palpable pubic bone is performed after careful shaving of the groins, external genitalia and pubic bone area, 10 minutes Betadine scrubbing and application of Povidon disinfectant left to dry.

The dorsal plane of the pubic bone and symphysis are exposed with continuous view on the periost, step by step cutting the suspensory ligament in the midline and in the depth and midline respecting the neurovascular bundle. In this region the corpora cavernosa separate to form the crurae, which both are mobilized from the lowest edge of the pubic bone for 1cm max. Both lateral suspensory ligaments are clearly visualized now and cut, leaving 2cm of tissue at the corporal side to fix the implant later. Both proximal corpora cavernosa are exposed by cutting the overlying fat in the midline again respecting the neurovascular bundle. Next step is removing laterally 1cm of fat on both sides to facilitate placing, suturing and covering the wedge in several separate layers.

Careful hemostasis before manual traction on the penis to judge the ideal size of the wedge. Definite choice of the soft solid medical silicon wedge and place it. Decision for optimal position of fixation and covering sutures with Prolene 1-0 to symphysis(4 stay sutures), the lateral suspensory ligaments remain(4 stay sutures) and multiple Vicryl 3-0 intermittent sutures to cover the wedge with several layers of fatty tissue first and subcutaneous tissue thereafter. The original V-incision is closed as V-Y of which the "leg"length of the Y will represent an impression of the length gain obtained by the implant. The V-Y skin defect will be closed with intracutaneous Vicryl Rapide 3-0. No drains will be left to minimize the risk of infection from the outside.

One or more embodiments of the present invention are described in the appended claims. Said claims also form part of this description.

## Claims

1. A penile implant comprising at least a first and second side wherein when implanted the first side faces the pubic symphisis and the second side faces the dorsal corpora cavernosa.

2. Penile implant according to claim 1 wherein the implant is substantially triangular in shape.

3. Penile implant according to claim 1 or 2 further comprising means to connecting the implant to at least the pubic symphisis and/or the corpora cavernosa.

4. Penile implant according to claim 3 wherein the means for connecting is a jacket of a mesh-work of fibers.

5. Penile implant according to one or more of the previous claims wherein the first and second side define an angle, said angle preferably is from 45 to 75 degrees.

6. Penile implant according to any one or more of the previous claims which is comprised of soft silicon, and preferably medical grade soft silicon.

7. Penile implant according to claim 6 wherein the implant consists of a solid body of soft silicon and a mesh work.

8. Penile implant according to claim 7 wherein said mesh work encompasses said solid body of soft silicon.

9. Penile implant according to one or more of the previous claims wherein the first side ranges from 3 to 5 cm.

10. Penile implant according to one or more of the previous claims wherein the second side ranges from 4 to 11 cm.

11. A kit comprising a number of penile implants, preferably at least 3, and more preferred at least 6, preferably said number of penile implants are of different size.

12. Method for manufacturing a penile implant comprising the steps of:
i) Providing a mold;
ii) Providing to the template soft silicon, preferably medical grade soft silicon;
iii) Removing said mold after a certain amount of time to obtain the penile implant.

13. Method according to the previous claim further comprising the step of providing to the implant obtained in step iii) a mesh-work of fibers, preferably said mesh work is in the form of a sheet or tape.

14. Penile implant according to one or more of claims 1-10 or a kit according to claim 11 for use in a cosmetic treatment, said cosmetic treatment is preferably penile lengthening or penile correction.

15. Penile implant according to one or more of claims 1-10 or a kit according to claim 11 for use in the prevention of the retraction of the corpora cavernosa.

16. Penile implant according to one or more of claims 1-10 or a kit according to claim 11 for use in combination with a prosthesis for penile erection.
